# EUROPEAN PATENT SPECIFICATION

(11) **EP 2 824 103 B1**
(45) Date of publication and mention of the grant of the patent: **27.04.2016**
(21) Application number: 14176353.2
(22) Date of filing: 09.07.2014
(51) Int. Cl.: C07D 401/14, A61K 31/4545, C07D 213/30, A61P 37/08, A61P 11/02

(54) **An improved process for the preparation of Rupatadine Fumarate**
Verbessertes Verfahren zur Herstellung von Rupatadinfumarat
Procédé amélioré de préparation de Rupatadine Fumarate

(30) Priority: 09.07.2013 IN MU23082013
(43) Date of publication of application: 14.01.2015
(73) Proprietor: Cadila Pharmaceuticals Limited, Bhat, Ahmedabad 382 210 (IN)
(72) Inventor: Modi, Rajiv Indravadan, Bhat Ahmedabad- 382210 Gujarat (IN); Gudaparthi Omprakash, Bhat Ahmedabad- 382210 Gujarat (IN); Chowdhary Anil Shankar, Bhat Ahmedabad- 382210 Gujarat (IN); Kumar Neeraj, Bhat Ahmedabad- 382210 Gujarat (IN); Patel Nilesh Govindbhai, Bhat Ahmedabad- 382210 Gujarat (IN); Soni Sanjay Kanhaiyalal, Bhat Ahmedabad- 382210 Gujarat (IN)
(74) Representative: Rigby, Barbara Nicole

(56) References cited:
- WO-A2-2006/114676
- RAJENDRA AGARWAL ET AL: "Expedient Synthesis of Rupatadine", SYNTHETIC COMMUNICATIONS, vol. 38, no. 1, 1 December 2007 (2007-12-01), pages 122-127, XP055149711, ISSN: 0039-7911, DOI: 10.1080/00397910701651276
- CARCELLER E ET AL: "Ä(3-Pyridylalkyl)piperidylideneÜbenzocycl ohepatypyridine derivatives as Dual Antagonists of PAF and Histamine", JOURNAL OF MEDICINAL CHEMISTRY, AMERICAN CHEMICAL SOCIETY, US, vol. 37, 1 January 1994 (1994-01-01), pages 2697-2703, XP003005099, ISSN: 0022-2623, DOI: 10.1021/JM00043A009

## Description

The following specification describes the nature of the invention, and the manner in which it is to be performed.

### FIELD OF THE INVENTION

The present invention provides an improved process for the preparation of Rupatadine Fumarate having structural Formula-1 and being substantially free from dimer and quaternary impurities.

### BACKGROUND OF THE INVENTION

Rupatadine Fumarate of structural Formula-1 chemically named as fumarate salt of 8-Chloro-11-[1-(5-methyl-pyridin-3-ylmethyl)-piperidin-4-ylidene]-6,11-dihydro-5H-benzo[5,6]-cyclohepta[1,2-b]pyridine is a potent orally active dual antagonist of platelet-activating factor (PAF) and effect through its interaction with histamine (H1) receptor.

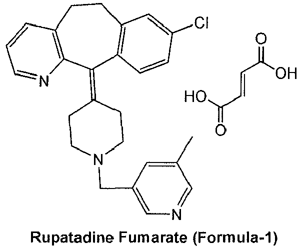

Various synthetic procedures for the preparation of Rupatadine Fumarate are disclosed in prior art.

EP0577957B1 discloses three different processes for the preparation of Rupatadine and its fumarate salt. In one of the process depicted as Scheme-1, discloses hydrolytic removal of the *N*-ethoxycarbonyl group of Loratadine having structural Formula-A to give Desloratadine having structural Formula-B, is *N*-acylated with 5-methylnicotinic acid using 1,3-dicyclohexylcabodiimide (DCC) and 1-hydroxybenzotriazole hydrate (HOBT) to give amide derivative of Formula-6, which further reacts with Phosphorus oxychloride (POCl₃) and Sodium borohydride (NaBH₄) to give Rupatadine Base. 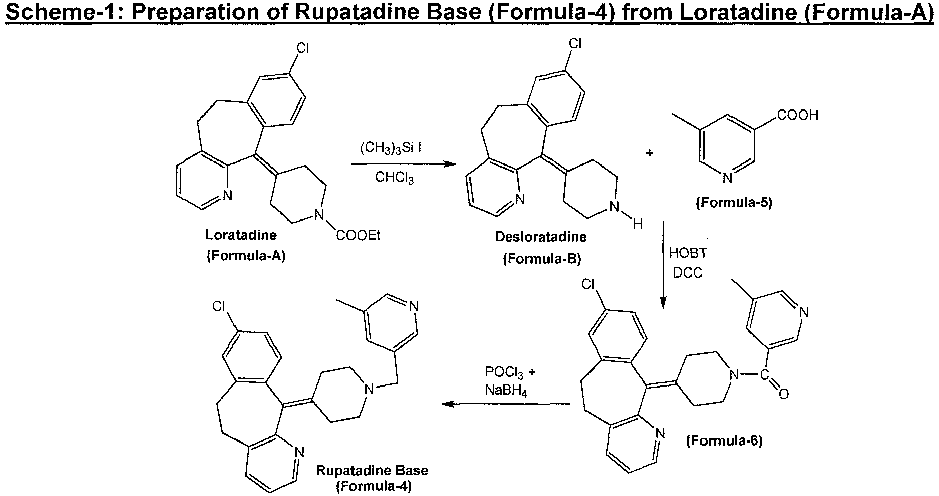 Rupatadine Base prepared as per scheme-1, is resulting following drawbacks:
a) Use of 1,3-dicyclohexylcabodiimide (DCC) and 1-hydroxybenzotriazole hydrate (HOBT), which generates dicyclohexyl urea as a byproduct and removal of this byproduct requires repeated purification which leads to reduced over all yield.
b) 5-methylnicotinic acid (Formula-5) generally contains substantial amount of 3,5-pyridine dicarboxylic acid (Formula-2A) which during the course of the reaction, forms dimer impurity (Formula-2B), which due to structural similarity is difficult to remove.

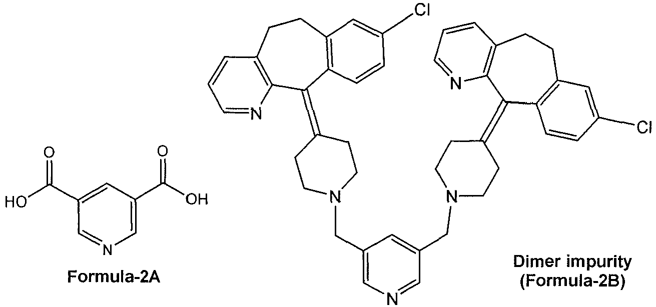

An another process for the preparation of Rupatadine is depicted in Scheme-2, discloses reaction of 3,5-lutidine with *N*-Bromosuccinimide (NBS) in Carbon tetrachloride (CCl₄) to give 3-Bromomethyl-5-methyl-pyridine - an intermediate which further reacts with Desloratadine in presence of TEA and DMAP to give Rupatadine Base of Formula-4. 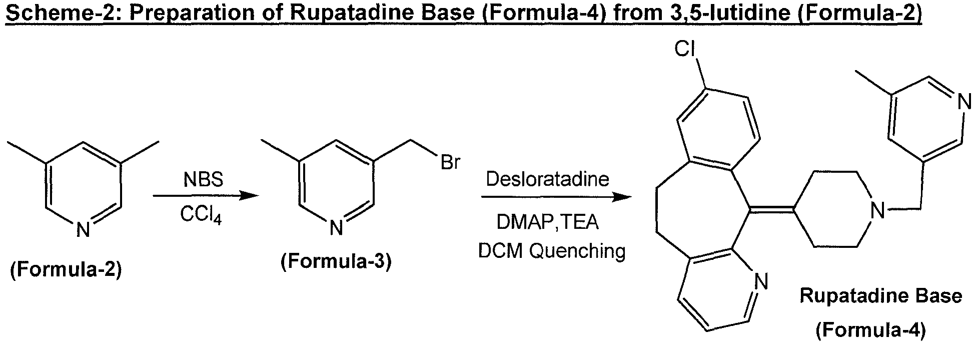 Rupatadine Base prepared as per scheme-2, is resulting following drawbacks:
a) bromination of 3,5-lutidine produces di-bromo, tri-bromo and tetra-bromo reactive impurities are formed, which subsequently forms the dimer impurity which is very difficult to separate from Rupatadine Fumarate.
b) carbon tetrachloride is a known potential carcinogenic solvent and is not use for commercial production.
c) In the condensation step of Rupatadine base preparation, 3-bromomethyl-5-methylpyridine is reacts further with Rupatadine base and forms quaternary impurity (Formula-2C). Its purification leads to substantial drop in yield.

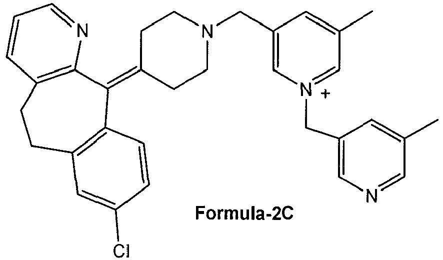

Another process for the preparation of Rupatadine is depicted as Scheme-3,5-methylnicotinic acid of Formula-5 is chlorinated with Phosphorus oxychloride (POCl₃) and condensed with Desloratadine to get compound of Formula-6, which on reduction using sodium borohydride (NaBH₄) results Rupatadine base. 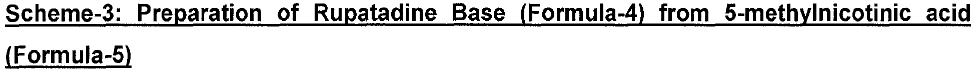 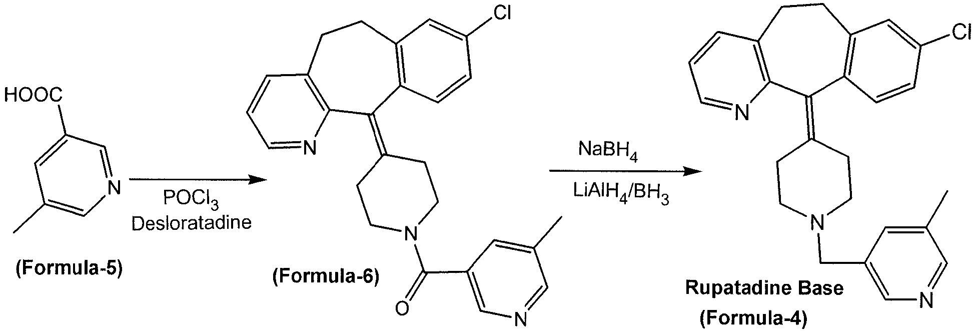 Rupatadine Base prepared as per scheme-3 results the following drawbacks:
a) there is no control on the formation of quaternary and dimer impurities leading to poor quality material.
b) use of hazardous LiAlH₄ or di-boran.

PCT publication WO2006114676 discloses a process for preparation of Rupatadine Fumarate (depicted as Scheme-4), which disclosed *N*-alkylation of Desloratadine with 3-bromomethyl-5-methyl pyridine (Formula-3) using a inorganic base and a phase transfer catalyst in biphasic solvent system. Rupatadine Fumarate is prepared from Rupatadine base using Fumaric acid in a mixture of methanol and ketonic solvent. 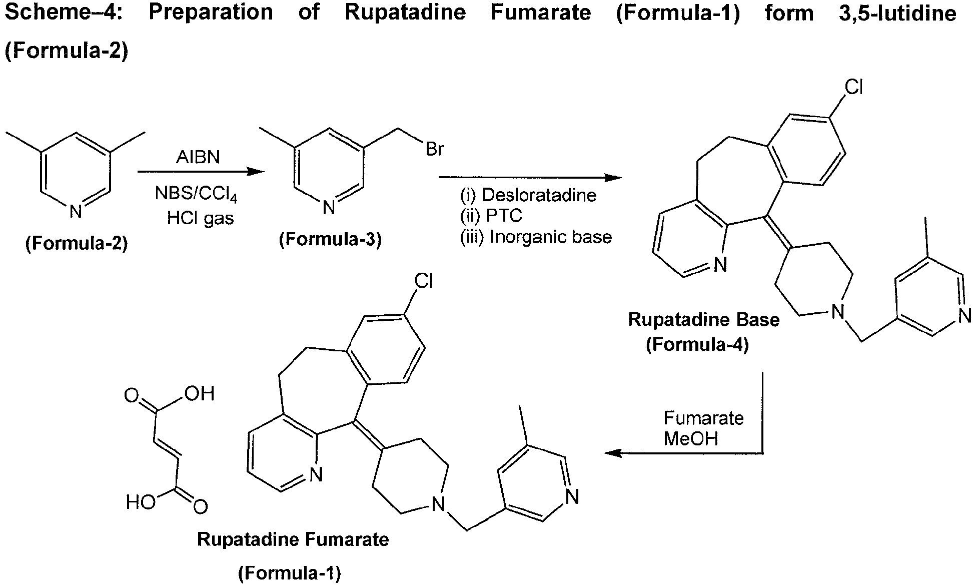 Rupatadine Base prepared as per scheme-4 is results the following drawbacks:
a) Intermediate Formula-3 is prepared via free radical reaction with a 19% conversion only. Further, various brominated products e.g. di-bromo, tri-bromo and tetra-bromo are forming and leading to dimer impurity formation in due course of further steps. Removal of dimer impurity is not possible via simple crystallization.
b) Isolation of intermediate Formula-3, which is lachrymatory and skin irritating in nature.

Indian patent application IN2102MUM2006 discloses a process for the preparation of Rupatadine Fumarate as depicted as Scheme-5, the process includes, esterification of 5-methyl nicotinic acid of Formula-7 followed by reduction which is further reduces to 5-methylpyridine-3-methanol of Formula-9 using LiAlH₄. The resulted intermediate is further reacted with thionyl chloride to furnish 5-methyl-3-pyridylmethyl chloride of Formula-10 as hydrochloride salt. Then it is treated with Desloratadine in the presence of base and di-methyl formamide (DMF) to give Rupatadine Base (Formula-4). Rupatadine Base is further treated with Fumaric acid to give Rupatadine Fumarate of Formula-1. 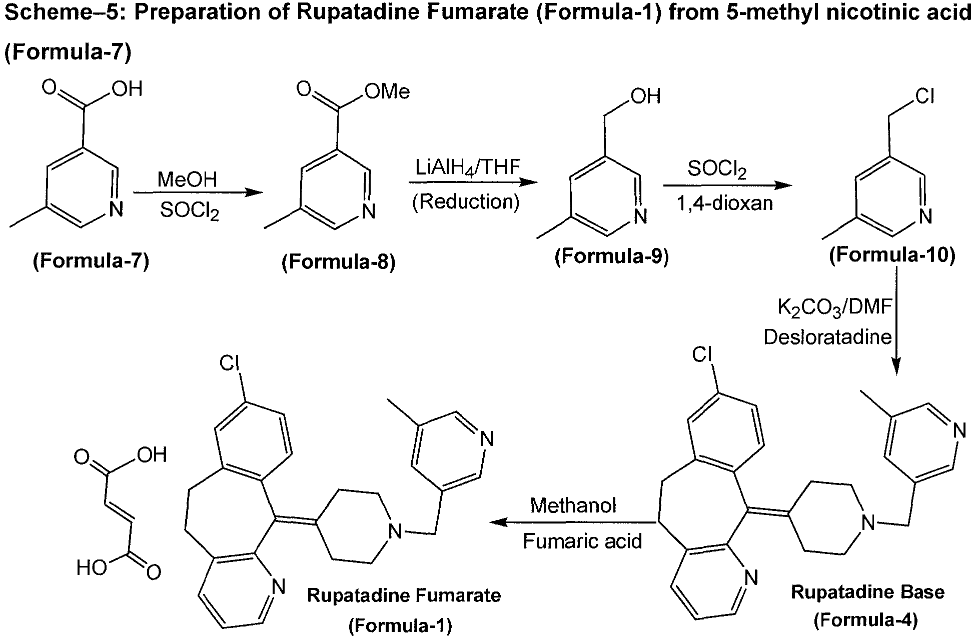 Rupatadine Base prepared by Scheme-5 results the following drawbacks:
a) Use of LiAlH₄
b) there is no control over formation of dimer and quaternary impurities.
c) isolation of intermediate Formula-10, which is lachrymatory and skin irritating in nature.

Indian patent application IN864/MUM/2006 discloses a process for the preparation of Rupatadine Fumarate which involves a reaction of 5-methyl nicotinic acid methyl ester of Formula-8 with Di-potassium phosphate (K₂HPO₄) and NaBH₄ in ethanol to provide 5-methylpyridine-3-methanol of Formula-9 which is further purified using IPA.HCl to get pure 5-methylpyridine-3-methanol in next step. Then it is reacted with para toluene sulfonyl chloride in presence of base in methylene dichloride (MDC) results intermediate of Formula-11. The intermediate of Formula-11 is reacted with Desloratadine in presence of K₂CO₃ and acetone to give Rupatadine base which on further treatment with fumaric acid in methanol to give Rupatadine Fumarate of Formula-1. 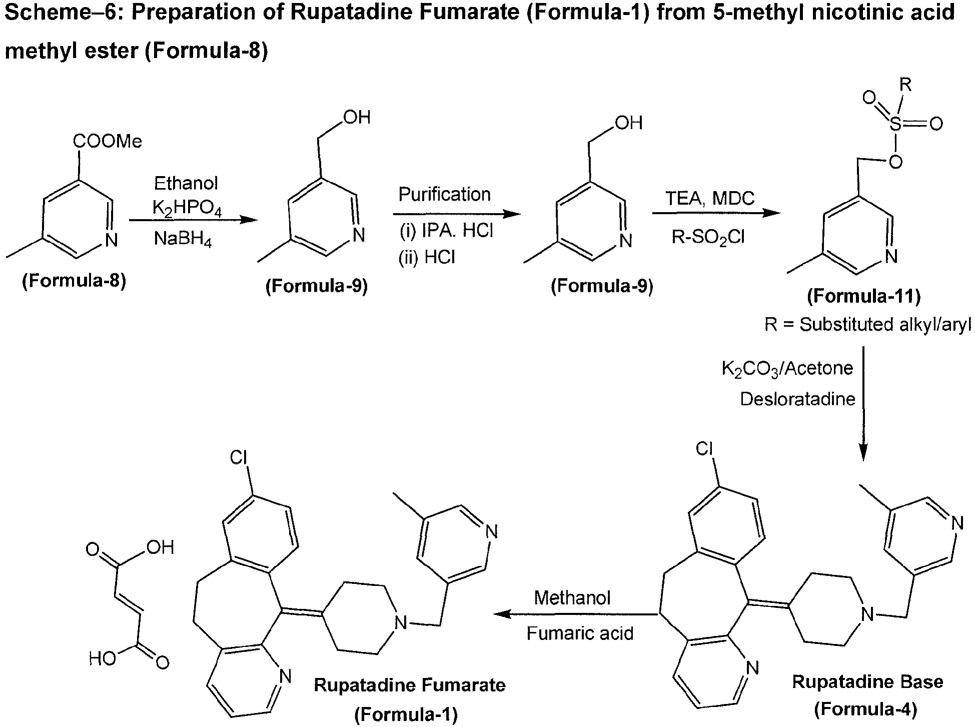 Rupatadine Base prepared by Scheme-6 has following drawbacks:
a) during the process, no control over formation of dimer and quaternary impurities.

The process reported in relevant prior art are non efficient to produce Rupatadine Fumarate of desired purity and yield. All of the reported process is lacking a control over impurity formation and its purification.

### SUMMARY OF THE INVENTION

A main objective of present invention is to provide an improved process for the preparation of Rupatadine Fumarate with reduced level of impurities.

Another objective of the present invention is to provide a process for the preparation of Rupatadine Fumarate with reduced level of dimer impurity.

Yet another objective of the present invention is to provide a process for the preparation of Rupatadine Fumarate with reduced level of quaternary impurity.

Yet another objective of present invention is to provide an improved process for the preparation of Rupatadine Fumarate which is substantially free from dimer and quaternary impurities.

Yet another objective of the present invention is to prepare Rupatadine by one pot conversion of 3-chloromethyl-5-methyl pyridine solution.

Yet another objective of the present invention is to prepare Rupatadine by condensation of 3-chloro-5-methylpyridine with Desloratadine using a phase transfer catalyst and an aqueous alkali in a biphasic system.

Yet another objective is to prepare 5-methylpyridine-3-methanol, substantially free from 3,5-pyridinedimethanol.

### DETAILED DESCRIPTION OF THE INVENTION

In accordance with the objective of present invention to provide an improved process for the preparation of Rupatadine Fumarate having reduced level of impurities (dimer and quaternary impurities) which in turn results the high yield of end product. The process for the preparation of Rupatadine Fumarate comprises:
A process for preparation of 5-methylpyridine-3-methanol comprising the steps of:
   a) reducing 5-methyl nicotinic acid alkyl ester containing 3,5-pyridine dicarboxy acid esters, using alkali metal borohydride and alkali metal alkoxide to give 5-methylpyridine-3-methanol and 3,5-pyridinedimethanol;
   b) washing with brine followed by layer separation to give 5-methyl-pyridine-3-methanol in organic phase;
   c) separating the organic phase and removing the organic solvent to obtain 5-methylpyridine-3-methanol, substantially free from 3,5-pyridinedimethanol.
The intermediate product obtained in step-c is preferably further reacted to obtained Rupatadine Fumarate as final product, which comprising steps of:
   d) chlorinating 5-methylpyridine-3-methanol to give 5-methyl-3-pyridinylmethyl chloride in aromatic hydrocarbons or ether;
   e) reacting solution of 5-methyl-3-pyridinylmethyl chloride with desloratadine using inorganic base and phase transfer catalyst to give Rupatadine.
The steps involved in the synthesis of Rupatadine Fumarate (Formula-1) according to a preferred embodiment of the process of present invention are depicted as scheme-7:

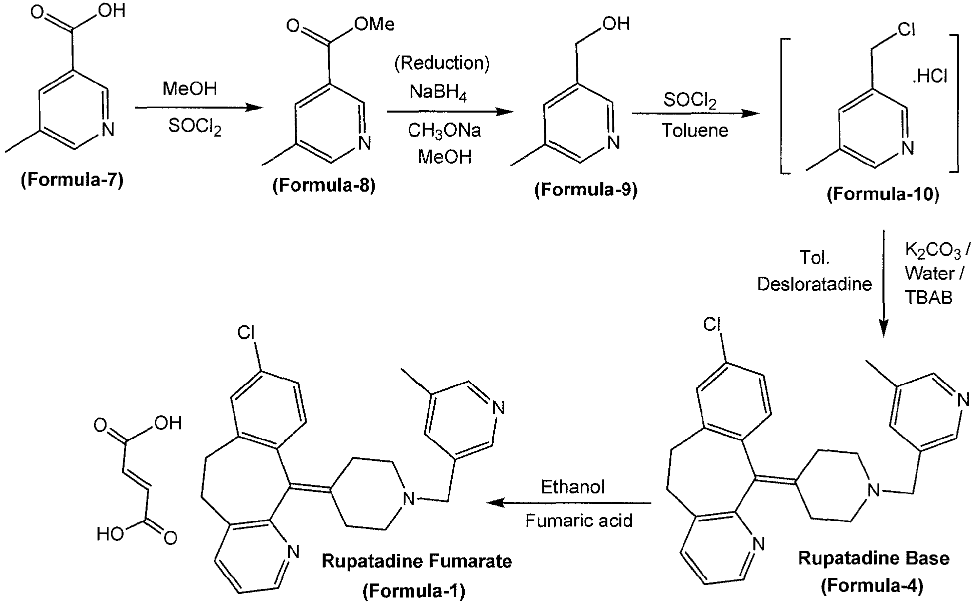

In a preferred embodiment, the present invention provides the process for preparation of Rupatadine base or Rupatadine Fumarate which is substantially free from dimer and quaternary impurities. The esterification of the acid (Formula-7) is preferably carried out with lower alcohol in presence of a thionyl chloride to produce ester of 5-Methyl-nicotinic acid (Formula-8). The lower alcohol is preferably selected from group consisting of methanol, ethanol and propanol. This process is preferably carried out under nitrogen atmosphere and the reaction mixture is preferably heated, most preferably to 60-65°C, preferably for 2-3 hours. After completion, which may be monitored by TLC, the alcohol may be removed, preferably by evaporation under reduced pressure. Water may be added and the pH may be adjusted to about 7 for example using ammonia. The reaction mass may be extracted using MDC. The organic layers may be combined and washed with brine, dired and filtered. The ester (Formula-8) can also be obtained by various methods known in the art. Thus, the process of the invention may optionally include the provision of Formula-8 via the steps discussed above.
5-methyl-3-pyridine carboxylic acid (Formula-7) contains sufficient amount of Pyridine-3,5-dicarboxylic acid (Formula-2A) which is generally produced by oxidation of 3,5-lutidine (Formula-2), which contains 0.5 to 7% of 3,5-dicarboxylic acid, hence it is not possible to prepare 5-methyl-3-pyridine carboxylic acid free from pyridine-3,5-dicarboxylic acid. Pyridine-3,5-dicarboxylic acid is a reactive impurity and it generates corresponding derivatives such as di-ester (Formula-2D), di-alcohol (Formula-2E) and di-chloro methyl pyridine derivative (Formula-2F) during Rupatadine synthesis.

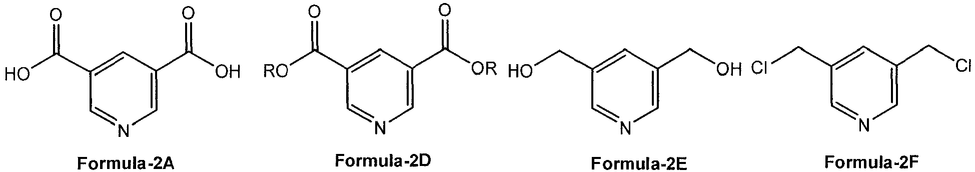

The di-chloro methyl pyridine derivative (Formula-2F) is very reactive and reacts with 2 molecules of Desloratadine to form corresponding dimer impurity (Formula-2B), which is not only undesired impurity but also reacts with Desloratadine and reduces it availability for reaction. Purification of dimer impurity from Rupatadine base or Rupatadine Fumarate is not possible due to its poor solubility. Hence, it is important to remove this impurity at earlier stage.

The term "substantially free of impurities" means less than 0.2% of total impurity as measured by area percentage HPLC., preferably less than 0.1 % of total impurities as measured by area percentage HPLC, most preferably less than detectable level of impurities as measured by area percentage HPLC.

The preparation of alcohol (Formula-9) can preferably be achieved by reduction of ester (Formula-8) by using with reducing agents like alkali metal borohydride such as sodium borohydride, potassium borohydride, lithium borohydride and/or zinc borohydride. Preferably it is sodium borohydride. The alkali metal borohydride is preferably used in the presence of a base as a catalyst like alkali metal alkoxide such as sodium methoxide, potassium methoxide, sodium ethoxide and/or potassium ethoxide. Preferably it is sodium methoxide. The base is preferably in a solvent, most preferably in alcohol or ether as a solvent. Particularly preferred is sodium borohydride in the presence of a base as a catalyst. Also preferred is sodium methoxide in alcohol or ether as a solvent.

The reduction is preferably carried out via formation of NaBH₃(OCH₃), preferably using CH₃ONa and NaBH₄. The preferred quantity of the alkali metal borohydride (preferably NaBH₄) is 1 to 3 mole equivalent more preferably 2 mole equivalent and most preferably is 1.7 mole equivalent. The preferred bases are metal alcoxide and the most preferred base is CH₃ONa. The preferred quantity of base is 0.1 to 1 mole equivalent more preferably 0.5 mole and most preferably is 0.2 mole. Solvents are preferably present and the preferred solvent for the reaction is selected from alcohols such as methanol, ethanol, isopropanol, n-butanol and ethers such as tetrahydrofuran, 2-methyl tetrahydrofuran, 1,2-di-methoxy ethane. The most preferred solvent is alcohol, most preferably methanol.

Preferably, on completion of the reaction, the solvent, e.g. methanol, may be removed by distillation. Water and MDC may be added. The pH of the reaction mixture may be adjusted, preferably to <3, more preferably to <2.5, for example using HCl. The aqueous layer may then be separated and the pH may be adjusted, preferably to 9-10, for example using NaOH, and the products may be extracted using a suitable solvent, preferably a chlorinated hydrocarbon such as MDC.

During reduction of 5-methyl nicotinic acid methyl ester using any reducing agent 0.5 to 5% of di-alcohol (Formula-2E) impurity is forming. And removal of this reactive impurity is not possible by crystallization. Surprisingly we have found that, the compound 3,5-pyridine - dimethanol product (di-alcohol) impurity can be selectively removed from the solution of 5-methyl nicotinic acid methyl ester with brine, preferably a saturated NaCl solution. Therefore the methods of the invention include the step b) of washing with brine. The brine may preferably be any salt in water solution. The salt is preferably sodium chloride (NaCl) but alternatively it may be potassium chloride (KCI), Lithium chloride (LiCI), Calcium chloride (CaCl2), magnesium chloride (MgCl2). The solution is most preferably saturated with salt, for example comprising about 360 grams of salt in one liter of water. However, solutions comprising less salt, for example up to 360 and no less than 100, 150, 200, 250, 300 or 320 grams of salt per litre of water are also contemplated. Alternatively viewed, the solution preferably comprises at least 10, 15, 20, 21, 22, 23, 24, 25, 26, 27, 28, 29 or 30% salt. The wash step may involve the addition of brine with stirring.

After the wash, the solvent (preferably MDC) layer may be separated, and the solvent may then be removed completely, preferably by distillation.

The preferred solvent is chlorinated hydrocarbon, hydrocarbon, preferably methylene dichloride.

**Table-1: HPLC results for Diol impurity level:**

| **S. No.** | **Diol impurity Before NaCl washing** | | **Diol impurity After NaCl washing** | |
|---|---|---|---|---|
| | **Rel. Area (%)** | **RRT** | **Rel. Area (%)** | **RRT** |
| 1 | 0.80 | 0.37 | 0.02 | 0.40 |
| 2 | 0.67 | 0.37 | 0.02 | 0.40 |
| 3 | 0.58 | 0.37 | ND | 0.40 |

From the above mentioned HPLC data in table-1, it is observed that after washing with sat. NaCl solution, the level of diol impurity decreases to 0.02 level to below detection level. i.e. in S. No.-1 & 2, maximum relative area of diol impurity before sat. NaCl washing is 0.80 % at 0.37 RRT, while after washing, the diol impurity relative area is 0.02% at 0.40 RRT. In S. No.-3, relative area of diol impurity before sat. NaCl washing is 0.58 % at 0.37 RRT, while after washing the diol impurity relative area is below detection level (ND) % at 0.40 RRT.

The chlorination of 5-methylpyridine-3-methanol (Formula-9) is preferably carried out using an aromatic hydrocarbon and a suitable chlorinating agent to produce 3-Chloromethyl-5-methyl-pyridine hydrochloride (Formula-10). A suitable solvent may be present and the most preferred solvent for the reaction is toluene or xylene. The chlorinating agent is preferably selected from PCl₅, PCl₃, POCl₃ and/or SOCl₂, most preferably SOCl₂. The reaction may preferably be carried out at 20-40°C and then be refluxed at about 60°C for about 2 hours. 3-Chloromethyl-5-methyl-pyridine hydrochloride (Formula-10) is preferably used *in situ* in Rupatadine base (Formula-4) preparation. The 3-Chloromethyl-5-methyl-pyridine hydrochloride (Formula-10) is preferably converted into free base using a base in reaction medium and organic layer was taken for next step without isolating 3-Chloromethyl-5-methyl-pyridine hydrochloride (Formula-10).

The Rupatadine base (Formula-4) is preferably prepared from reaction of 3-Chloromethyl-5-methyl-pyridine hydrochloride (Formula-10) with Desloratadine in the presence of a phase transfer catalyst and a base in aromatic hydrocarbon and water as a solvent. The most preferred hydrocarbon for the reaction is toluene or xylene. The phase transfer catalyst used may for example be triethyl benzyl ammonium chloride, tetrabutyl ammonium bromide, tetrabutyl ammonium hydrogen sulfate, preferably tetrabutyl ammonium bromide. The base used in the reaction is preferably NaOH, KOH, LiOH, Ca(OH)₂, Ba(OH)₂, Na₂CO₃, K₂CO₃, Li₂CO₃, CaCO₃, KHCO₃, NaHCO₃ and their like, more preferable is K₂CO₃. The reaction may preferably be refluxed at about 65-70°C for about 2 hours.
During Rupatadine base preparation the Rupatadine base further reacts with 3-Chloromethyl-5-methyl-pyridine hydrochloride (Formula-10) to generate quaternary impurity (Formula-2C). Surprisingly, it has been observed that the quaternary impurity (Formula-2C) is insoluble in aromatic hydrocarbon.

Thus, in a preferred embodiment, on completion of the reaction the lower aqueous layer and impurity layer may be removed, for example using a suitable solvent such as toluene. Potassium dihydrogen phosphate may be added, e.g. with stirring for 1 hr. The layers may be allowed to separate followed by addition of charcoal in the reaction mass and the reaction mass may be heated, e.g. at 40-45°C for about 2 hrs. Then the reaction product may be filtered e.g. through a hyflow bed and the solvent may be removed, e.g. by evaporation under reduce pressure. The oily mass may then be dissolved in SDS.

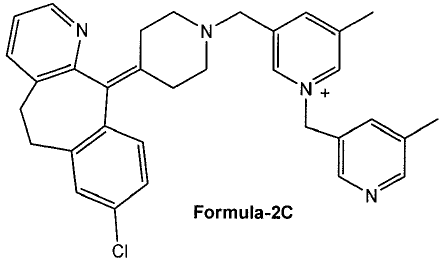

The Rupatadine Fumarate (Formula-1) is preferably produced from Rupatadine Base (Formula-4) using Fumaric acid in ethanol. The product may then be filtered, washed with ethanol, dried and recrystallized, for example using ethanol.
The present invention is illustrated with following examples.

### Example-1: Synthesis of 5-Methyl-nicotinic acid methyl ester (Formula-8) from 5-Methyl-nicotinic acid (Formula-7):

Under nitrogen atmosphere, Thionyl chloride (110.0 ml; 1.50 moles) is added drop wise at 20-25°C to a suspension of 5-methyl nicotinic acid (100.0g; 0.73 moles) in methanol (500.0 ml) kept under nitrogen atmosphere at 20-25°C and heated the reaction mixture to 60-65°C for 2-3 hrs. On completion of the reaction is monitored by TLC. After completion of the reaction methanol is evaporated under reduced pressure to get a residue. Chilled water (150.0 ml) is added to the residue and pH of the reaction mass is adjusted to 7.0 using ammonia. The reaction mass is extracted using MDC (250.0 ml x 2). The organic layers were combined and washed with brine (150.0 ml), dried over anhydrous sodium sulfate and filtered. The filtrate is evaporated under reduced pressure to obtain the title intermediate compound (150.0 g; Yield=96%) HPLC purity- 99.00%

### Example-2: Synthesis of 5-methylpyridine-3-methanol (Formula-9) from 5-Methyl-nicotinic acid methyl ester (Formula-8):

5-methyl nicotinic acid methyl ester (Formula-8) (100.0 gm, 0.66 moles) in methanol (400 ml) and stir the reaction mass. Then add Sodium borohydride (36.72gm, 0.96 moles) and Sodium methoxide (10.68 gm, 0.2 moles) are added under continuously stirring. On completion of reaction, methanol is distilled the methanol and then MDC and water to the reaction mixture. pH of reaction mixture is adjusted to <2.5 by using HCl aqueous layer is separated and pH is adjusted to 9-10 using NaOH and then product is extracts using MDC. Saturated brine solution is added with stirring followed by separating MDC layer. MDC is distilled completely to obtained title intermediate compound. (64-68.0 g; Yield=79-84%)

### Example-3: Synthesis of Rupatadine base (Formula-4) from 5-methylpyridine-3-methanol (Formula-9):

Take 5-methylpyridine-3-methanol (Formula-9, 51 gm) in toluene (255 ml) and add slowly thionyl chloride (36.5 ml) between 20-40°C. On completion of thionyl chloride addition, reflux the reaction mixture at 60°C for 2 hrs. On the completion of reaction toluene is distilled toluene under vacuum. Toluene (250 ml) and water (25 ml) added to the reaction mixture. Stir it between below 20°C temperature. Adjust the pH by using 35% NaOH solution and again stir the reaction to separate the layers. Desloratadine (95g) is added to the in the separated organic layers at 25-30°C. Then add Potassium carbonate (100g), TBAB (5g) and water (200 ml) is added to the reaction mass followed by reflux at 65-70°C for 2 hrs.

On the completion of reaction, separate lower aq. Layer and impurity layer using toluene. 10% solution of potassium dihydrogen phosphate (100 ml) is added with stirring for 1 hr. The layers are allowed to separate followed by addition of charcoal in the reaction mass and heat the reaction mass at 40-45°C for 2 hrs. Then filter through hyflow bed and evaporate solvent under reduce pressure. Followed by dissolving the oily mass in SDS (300 ml).

### Example4: Synthesis of Rupatadine Fumarate (Formula-1) from Rupatadine base (Formula-4):

Take ethanol (300 ml) in RBF and add slowly add fumaric acid in it with stirring the reaction mixture. Allowed the reaction mixture to reflux at 70-80°C for 1 hr. The solution of Rupatadine base of Formula-4 in SDS is added to reaction mixture during 30 minutes. The temperature of reaction mixture is maintained for 3 hrs. On completion of reaction, the filter the product and wash with ethanol. Dry the solid and recrystallized with the product using ethanol.

Further embodiments of the invention are set out below
1. A process for preparation of 5-methylpyridine-3-methanol comprising steps:
   a) reducing 5-methyl nicotinic acid alkyl ester containing 3,5-pyridine dicarboxy acid esters, using alkali metal borohydride, alkali metal alkoxide to give 5-methyl-pyridine-3-methanol and 3,5-pyridinedimethanol;
   b) washing with brine followed by layer separation to give 5-methyl-pyridine-3-methanol in organic phase;
   c) separating the organic phase and removing the organic solvent to obtain 5-methylpyridine-3-methanol, substantially free from 3,5-pyridinedimethanol.
2. The process as described in embodiment 1, wherein alkali metal borohydride is sodium borohydride, potassium borohydride or lithium borohydride and alkali metal alkoxide is sodium methoxide, potassium methoxide, sodium ethoxide and potassium ethoxide.
3. The process of preparing Rupatadine by reacting product obtained in embodiment 1 in steps,
   a) chlorinating 5-methylpyridine-3-methanol to give 5-methyl-3-pyridinylmethyl chloride in aromatic hydrocarbons or ether;
   b) reacting solution of 5-methyl-3-pyridinylmethyl chloride with desloratadine using inorganic base and phase transfer catalyst to give Rupatadine.
4. The process as described in embodiment 3, wherein the inorganic base is selected from alkali hydroxide, alkali carbonates or alkali bicarbonate.
5. The process as described in embodiment 3, wherein the phase transfer catalyst is tetrabutyl ammonium bromide, tetrabutyl ammonium chloride.
6. The process as described in embodiment 3, wherein the aromatic hydrocarbon is selected from toluene or xylene, the ether is methyl tert-butyl ether or di-isopropyl ether.
7. The process as described in embodiment 3, wherein reaction is yields Rupatadine free of quaternary impurity.
8. Rupatadine prepared as per the process claimed in embodiment 3 wherein Rupatadine contains the real area percentage of dimer or quaternary impurity less than 0.1 % or not detectable.

## Claims

1. A process for the preparation of 5-methylpyridine-3-methanol comprising the steps:
a) reducing 5-methyl nicotinic acid alkyl ester containing 3,5-pyridine dicarboxy acid esters, using alkali metal borohydride, alkali metal alkoxide to give 5-methylpyridine-3-methanol and 3,5-pyridinedimethanol;
b) washing with brine followed by layer separation to give 5-methyl-pyridine-3-methanol in organic phase;
c) separating the organic phase and removing the organic solvent to obtain 5-methylpyridine-3-methanol, substantially free from 3,5-pyridinedimethanol.

2. The process as claimed in claim 1, wherein the alkali metal borohydride is selected from sodium borohydride, potassium borohydride and/or lithium borohydride and the alkali metal alkoxide is selected from sodium methoxide, potassium methoxide, sodium ethoxide and/or potassium ethoxide.

3. A process of preparing Rupatadine, comprising preparing 5-methylpyridine-3-methanol via the process of claim 1 or 2 and further
i) chlorinating said 5-methylpyridine-3-methanol to give 5-methyl-3-pyridinylmethyl chloride in aromatic hydrocarbons or ether; and
ii) reacting said solution of 5-methyl-3-pyridinylmethyl chloride with desloratadine using an inorganic base and a phase transfer catalyst to give Rupatadine.

4. The process of any one of claims 3, wherein the inorganic base is selected from alkali hydroxide, alkali carbonates or alkali bicarbonate.

5. The process of any one of claims 3 to 4, wherein the phase transfer catalyst is tetrabutyl ammonium bromide or tetrabutyl ammonium chloride.

6. The process of any one of claims 3 to 5, wherein the aromatic hydrocarbon is selected from toluene or xylene, or the ether is methyl tert-butyl ether or di-isopropyl ether.

7. The process of any one of claims 3 to 6, wherein the reaction yields Rupatadine free of quaternary impurity.

## Patentansprüche

1. Verfahren zur Zubereitung von 5-Methylpyridin-3-Methanol, umfassend die Stufen:
a) Reduzieren eines 5-Methyl-Nikotinsäureesters, der 3,5-Pyridin-Dicarboxysäureester enthält, unter Verwendung von Alkali-Metallborhydrid, Alkali-Metallalkoxid, um 5-Methyl-Pyridin-3-Methanol und 3,5-Pyridindimethanol zu ergeben,
b) Waschen mit Lauge, das von einer Schichtentrennung gefolgt wird, um 5-Methyl-Pyridin-3-Methanol in einer organischen Phase zu ergeben,
c) Trennen der organischen Phase und Entfernen des organischen Lösungsmittels, um 5-Methylpyridin-3-Methanol zu erhalten, das im Wesentlichen von 3,5-Pyridindimethanol frei ist.

2. Verfahren gemäß Anspruch 1, bei dem das Alkali-Metallborhydrid aus Natrium-Borhydrid, Kalium-Borhydrid und / oder Lithiumborhydrid ausgewählt ist und das Alkali-Metallalkoxid aus Natrium-Methoxid, Kalium-Methoxid, Natrium-Ethoxid und / oder Kalium-Ethoxid ausgewählt ist.

3. Verfahren zur Zubereitung von Rupatadin, umfassend die Zubereitung von 5-Methylpyridin-3-Methanol über das Verfahren gemäß Anspruch 1 oder 2 und weiterhin
i) Chloren des genannten 5-Methylpyridin-3-Methanol, um 5-Methyl-3-Pyridinylmethyl-Chlorid in aromatischen Kohlenwasserstoffen oder Äther zu ergeben; und
ii) Reagieren der genannten Lösung aus 5-Methyl-3-Pyridinylmethyl-Chlorid mit Desloratadin unter Verwendung einer anorganischen Basis und eines Phasenübertragungskatalysators, um Rupatadin zu ergeben.

4. Verfahren gemäß Anspruch 3, bei dem die anorganische Basis aus Alkali-Hydroxid, Alkali-Karbonaten oder Alkali-Bikarbonaten ausgewählt ist.

5. Verfahren gemäß Anspruch 3 oder 4, bei dem der Phasenübertragungskatalysator Tetrabutyl-Ammomiumbromid oder Tetrabutyl-Ammomiumchlorid ist.

6. Verfahren gemäß einem der Ansprüche 3 bis 5, bei dem der aromatische Kohlenwasserstoff aus Toluol oder Xylol ausgewählt ist oder der Äther Methyl-tert-Butyläther oder di-Isopropyläther ist.

7. Verfahren gemäß einem der Ansprüche 3 bis 6, bei dem die Reaktion Rupatatin ergibt, das von quaternärer Unreinheit frei ist.

## Revendications

1. Processus pour la préparation de 5-méthylpyridine-3-méthanol comprenant les étapes de :
a) réduction de l'ester alkylique d'acide 5-méthyle nicotinique contenant des esters d'acide 3,5-pyridine dicarboxy, en utilisant du borohydrure de métal alcalin, de l'alcoxyde de métal alcalin pour donner du 5-méthylpyridine-3-méthanol et du 3,5-pyridinediméthanol ;
b) lavage avec de la saumure suivi par une séparation de couche pour donner du 5-méthyl-pyridine-3-méthanol dans une phase organique ;
c) séparation de la phase organique et enlèvement du solvant organique pour obtenir du 5-méthylpyridine-3-méthanol, sensiblement exempt de 3,5-pyridinediméthanol.

2. Processus selon la revendication 1, dans lequel le borohydrure de métal alcalin est sélectionné à partir de borohydrure de sodium, borohydrure de potassium et/ou borohydrure de lithium et l'alcoxyde de métal alcalin est sélectionné à partir de méthoxyde de sodium, méthoxyde de potassium, éthoxyde de sodium et/ou éthoxyde de potassium.

3. Processus de préparation de Rupatadine, comprenant la préparation de 5-méthylpyridine-3-méthanol via le processus selon la revendication 1 ou 2 et en outre
i) la chloration dudit 5-méthylpyridine-3-méthanol pour donner du chlorure de 5-méthyl-3-pyridinylmethyle dans des hydrocarbures aromatiques ou de l'éther ; et
ii) la réaction de ladite solution de chlorure de 5-méthyl-3-pyridinylméthyle avec de la desloratadine en utilisant une base inorganique et un catalyseur à transfert de phase pour donner de la Rupatadine.

4. Processus selon n'importe laquelle des revendications 3, dans lequel la base inorganique est sélectionnée à partir d'hydroxyde alcalin, des carbonates alcalins ou du bicarbonate alcalin.

5. Processus selon n'importe laquelle des revendications 3 à 4, dans lequel le catalyseur à transfert de phase est du bromure d'ammonium tétrabutylique ou du chlorure d'ammonium tétrabutylique.

6. Processus selon n'importe laquelle des revendications 3 à 5, dans lequel l'hydrocarbure aromatique est sélectionné à partir de toluène ou de xylène, ou l'éther est de l'éther méthylique de tert-butyle ou de l'éther de diisopropyle.

7. Processus selon n'importe laquelle des revendications 3 à 6, dans lequel la réaction donne de la Rupatadine sans impureté quaternaire.
